(19) Europäisches Patentamt
European Patent Office
Office européen des brevets

(11) **EP 3 094 968 B1**

(12) **EUROPEAN PATENT SPECIFICATION**

(45) Date of publication and mention
of the grant of the patent:
**09.12.2020 Bulletin 2020/50**

(51) Int Cl.:
**G01N 30/26** (2006.01)    **G01N 30/72** (2006.01)
**G01N 33/48** (2006.01)    **G01N 33/68** (2006.01)
**G01N 33/50** (2006.01)

(21) Application number: **15737286.3**

(22) Date of filing: **15.01.2015**

(86) International application number:
**PCT/US2015/011645**

(87) International publication number:
**WO 2015/109116 (23.07.2015 Gazette 2015/29)**

(54) **METABOLIC SCREENING FOR GESTATIONAL DIABETES**

METABOLISCHES SCREENING FÜR SCHWANGERSCHAFTSDIABETES

DÉPISTAGE MÉTABOLIQUE DU DIABÈTE GESTATIONNEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.01.2014 US 201461927657 P**

(43) Date of publication of application:
**23.11.2016 Bulletin 2016/47**

(73) Proprietor: **The Regents of the University of
California
Oakland, CA 94607-5200 (US)**

(72) Inventor: **KOOS, Brian J.
Los Angeles, California 90095 (US)**

(74) Representative: **FRKelly
27 Clyde Road
Dublin D04 F838 (IE)**

(56) References cited:
**US-A1- 2010 197 028    US-A1- 2011 123 986
US-A1- 2012 197 539**

- **HONGYUN WANG ET AL: "Measurement of 1- and 3-methylhistidine in human urine by ultra performance liquid chromatography-tandem mass spectrometry", CLINICA CHIMICA ACTA, vol. 413, no. 1-2, 1 January 2012 (2012-01-01), pages 131-138, XP055403299, AMSTERDAM, NL ISSN: 0009-8981, DOI: 10.1016/j.cca.2011.09.007**
- **DIAZ ET AL.: 'Second trimester maternal urine for the diagnosis of trisomy 21 and prediction of poor pregnancy outcomes' JOURNAL OF PROTEOME RESEARCH vol. 12, no. 6, 2013, pages 2946 - 2957, XP055185586**
- **PAPPA ET AL.: 'Gestational diabetes exhibits lack of carnitine deficiency despite relatively low carnitine levels and alterations in ketogenesis' THE JOURNAL OF MATERNAL-FETAL AND NEONATAL MEDICINE vol. 17, no. 1, 2005, pages 63 - 68, XP008184360**
- **SACHSE ET AL.: 'Metabolic changes in urine during and after pregnancy in a large, multiethnic population-based cohort study of gestational diabetes' PLOS ONE vol. 7, no. 12, 2012, page E52399, XP055214437**
- **DE SEYMOUR ET AL.: 'Early pregnancy metabolite profiling discovers a potential biomarker for the subsequent development of gestational diabetes mellitus' ACTA DIABETOLOGICA vol. 51, no. 5, 2014, pages 887 - 890, XP035398864**

**Description**

TECHNICAL FIELD OF THE INVENTION

The present invention relates generally to detection, diagnosis, and monitoring of diabetes mellitus, including gestational diabetes. The invention more specifically pertains to use of metabolic markers that can be detected in urine to screen for diabetes.

BACKGROUND OF THE INVENTION

The identification of gestational diabetes mellitus (GDM) currently involves either (1) universal screening by a 2-h, 75-g oral glucose tolerance test (OGTT) or a 1-h, 50-g oral glucose challenge test (OGCT) followed by a 3-h, 100-g oral glucose tolerance test (OGTT) for those with a positive OGCT, or (2) selective screening of high-risk groups based on age, race/ethnicity, body mass index (BMI), and family history. Venapuncture is required for both the OGCT and OGTT. The National Institutes of Health Consensus Development Conference Statement provides the current standards for diagnosing GDM (Obstet Gynecol 122:358-370, 2013). The use of glucose challenges to screen and diagnose GDM is limited by a lack of reproducibility and accuracy, excessive time involved, late gestational age (24-28 weeks of gestation) of testing, nausea, and the discomfort of venapunture (Brody et al. Obstet Gynecol 2003;101:380-392; Blayo. Diabetes Metab 2004;30:575-580; Sacks et al. Am J Obstet Gynecol 1989;161:642-645; Hanna, Diabet Med 2002:19:351-358).

The usefulness of serum/plasma markers for GDM have been examined, such as fasting glucose and insulin, C-reactive protein, sex hormone-binding globulin, adiponectin, and protein-associated plasma protein A (Smirnakis, et al. Am J Obstet Gynecol 2007;196:410e1-410e7; Georgiou et al., Acta Diabetol 2008;45:157-165; Kulaksizglu S. et al. Gynecol Endocrinol 2013;29:137-140). While significant differences have been found in those with GDM, these measurements lack sensitivity and specificity compared to the standard OGCT and OGTT methods.

Wang et al. (Clinica Chimica Acta, 2012; 413, no. 1-2, pages 131 - 138) discloses the measurement of 1- and 3-methylhistidine in human urine by ultra performance liquid chromatography-tandem mass spectrometry. US2012/197539 concerns a method for assessing patient health is provided using metabolomics. The method comprises providing a bodily fluid or tissue sample from a subject, collecting a metabolic profile from the bodily fluid or tissue sample and comparing the metabolic profile to a reference profile, wherein the preferred bodily fluid is urine. Diaz et al. (Journal of Proteome Research, 2013, 12, no. 6, pages 2946 - 2957) looks at second trimester maternal urine for the diagnosis of trisomy 21 and the prediction of poor pregnancy outcomes.

There is a need to identify improved markers for gestational diabetes. There is also a need for methods of detecting susceptibility to diabetes.

SUMMARY OF THE INVENTION

The invention provides a method of screening for susceptibility to diabetes in a subject. The method comprises measuring the amount of a metabolic marker present in a test sample of urine obtained from the subject, and comparing the amount of the metabolic marker present in the test sample to a control sample. The method further comprises identifying a subject as susceptible to, or having, diabetes if the amount of marker present in the test sample is increased or decreased relative to the control sample. Metabolic markers to be measured can be selected from the group consisting of the markers listed in Table 1. According to the invention, the metabolic marker comprises 1-methylhistidine. According to the invention, the, or each, metabolic marker concentration is normalized with respect to a selected normalizing marker selected from creatinine or osmolality. In one embodiment, the measuring comprises chromatography or spectrometry. The chromatography can be gas or liquid chromatography. The spectrometry can be, for example, mass spectrometry.

In one embodiment, the subject is pregnant, and the screening is for gestational diabetes mellitus (GDM). The method is typically performed when the subject is at least 6 weeks pregnant. The method is performed when the subject is 6-17 weeks pregnant, typically the method is performed when the subject is 6-14 weeks pregnant.

Testing can also be performed later in pregnancy, such as at about 22-30 weeks gestation, at term, or postpartum. The method can also be used in other populations as a general method of screening for diabetes and susceptibility to diabetes, such as type 2 diabetes mellitus.

The invention also provides a method of screening for susceptibility to diabetes in a subject, wherein the method comprises measuring the amount of at least two metabolic markers present in a test sample of urine obtained from the subject. The method further comprises comparing the amount of the metabolic markers present in the test sample to a control sample, and identifying a subject as susceptible to diabetes if the amount of the markers present in the test sample is increased or decreased relative to the control sample. Similarly, a method of detecting diabetes in a subject is disclosed. The method comprises measuring the amount of a metabolic marker present in a test sample obtained from the subject; and comparing the amount of the metabolic marker present in the test sample to a control sample. A subject is identified as having diabetes if the amount of marker present in the test sample is increased or decreased relative to the control sample. The metabolic markers are selected from the group consisting of the markers listed in Table 1. The markers can be selected from the group of markers listed in Table 2. The markers can be selected from the group of markers listed in Table 3. According to the invention, the metabolic markers comprise 1-methylhistidine and at least one marker selected from the group consisting of: alpha-CEHC glucuronide, anserine, methylsuccinate, xylonate, tigyl carnitine, quinolinate, pro hydroxy pro, 2-hydroxyisobutyrate, N-acetylthreonine, carnitine, and trigonelline. According to the invention, the, or each, metabolic marker concentration is normalized with respect to a selected normalizing marker selected from creatinine or osmolality.

In some embodiments of the invention, two markers selected from those described herein are measured. In other embodiments, three or more markers are measured. Additional markers can be used to improve the screening and detection, including any combination of two, three, four, five, six, seven, or more of the metabolic markers described herein. A combination of 8-11 markers can be used together for screening. Disclosed combinations of the metabolic markers include:

Anserine and methylsuccinate;
Sucrose and 1-methylhistidine;
Xylonate and tiglyl carnitine,
Quinolinate and pro hydroxy pro,
N-acetylthreonine and carnitine,
Trigonelline and sucrose;
Trigonelline and alpha-CEHC glucuronide;
Pyroglutamine and 2-hydroxyisobutyrate;
Methylsuccinate, anserine and 1-methylhistidine;
Methylsuccinate, sucrose and 1-methylhistidine;
Sucrose, anserine and 1-methylhistidine;
Adipate, cystathionine, and cytidine;
Pyroglutamine, anserine, and 2-hydroxyisobutyrate;
Anserine, cytidine, and cystathionine;
Adipate, pyroglutamine, and cystidine; and
Methylsuccinate, sucrose, anserine, and 1-methylhistidine.

Other disclosed combinations of metabolic markers selected from the group consisting of: 1-methylhistidine, 2-hydroxyisobutyrate, 3-(3-hydroxyphenyl)proprionate Itaconate, 3-fucosyllactose, 3-hydroxy-3-methylglutarate, 5-methylthioadenosine, Acetylcarnitine, Adipate, Agmatine, Alpha-CEHC glucuronide, Anserine, Carnitine, Cystathionine, Cytidine, Dihydrobiopterin, Galactose, Gamma-CEHC glucuronide, Gluconate, Glutamate, Glutarate, Glycocholate, Homoserine, Hydroxyphenylacetate, Lactose, Leucine, Methylsuccinate, N-acetylarginine, N-acetylthreonine, Nicotinate, Pro-hydroxy-pro, Pyroglutamine, Quinolinate, Scyllo-inositol, Sorbose, Sucrose, Thymine, Tigyl carnitine, Trigonelline, and Xylonate are also contemplated.

According to the invention, the marker can comprise 1-methylhistidine and any combination of one or more of the markers selected from the group consisting of: alpha-CEHC glucuronide, anserine, methylsuccinate, xylonate, tigyl carnitine, quinolinate, pro hydroxy pro, 2-hydroxyisobutyrate, N-acetylthreonine, carnitine, trigonelline, 3-fucosyllactose, 3-(3-hydroxyphenyl)proprionate, itaconate, and sucrose.

According to the invention, the combination of markers are:

- Sucrose and 1-methylhistidine;
- Methylsuccinate, anserine and 1-methylhistidine;
- Methylsuccinate, sucrose and 1-methylhistidine;
- Sucrose, anserine and 1-methylhistidine; and
- Methylsuccinate, sucrose, anserine, and 1-methylhistidine.

According to the invention, the combination of markers are: adipate, methylsuccinate, pyroglutamine, cytidine, 1-methylhistidine, 2-hydroxyisobutyrate, cystathionine, sucrose and anserine; or the combination of markers are: adipate, methylsuccinate, pyroglutamine, cytidine, 1-methylhistidine, 2-hydroxyisobutyrate, cystathionine, sucrose, glutamate, galactose, 3-hydroxy-3-methylglutarate, and anserine.

DETAILED DESCRIPTION OF THE INVENTION

The invention described herein is based on the discovery that certain metabolic markers can be used to detect, diagnose and monitor diabetes, including gestational diabetes mellitus, as well as to guide in the monitoring and selection of treatment. Testing early in pregnancy is desirable, as the early initiation of counseling and therapeutic measures can be reasonably expected to have the most beneficial effects in reducing perinatal morbidity. The availability of an accurate test that can be used in early pregnancy provides an important advance in the screening and diagnosis of GDM. This noninvasive test has major advantages in that it is painless and the sample is easily collected by medical assistants. The gravida's urine can be collected randomly at a routine prenatal visit and can be sent to a commercial laboratory for analysis. The collection does not involve a highly trained, expensive nurse or phlebotomist, as is required for blood sampling. This additional advantage will allow GDM testing in clinics with restricted resources for patient care. Another advantage is that a single urine test could replace GDM screening by the 1-h, 50-g OGCT and offer sufficient accuracy to eliminate the need for the painful, time-consuming 2-h, 75-g OGTT or 3-h 100-g OGTT.

Definitions

All scientific and technical terms used in this application have meanings commonly used in the art unless otherwise specified. As used in this application, the following words or phrases have the meanings specified.

As used herein, a "sample" from a subject means a specimen obtained from the subject that contains urine, blood, serum, saliva, or other bodily fluid.

As used herein, the term "subject" includes any human or non-human animal. The term "non-human animal" includes all vertebrates, e.g., mammals and non-mammals, such as non-human primates, horses, sheep, dogs, cows, pigs, chickens, amphibians, reptiles, rodents etc.

As used herein, a "control" sample means a sample that is representative of normal measures of the respective analyte. The sample can be an actual sample used for testing, or a reference level or range, based on known normal measurements of the corresponding analyte.

As used herein, "a" or "an" means at least one, unless clearly indicated otherwise.

Methods

The invention discloses a method of screening for susceptibility to diabetes in a subject. The invention provides a method particularly useful for identifying susceptibility to gestational diabetes mellitus (GDM) during early pregnancy, including during the first trimester of pregnancy as well as later in pregnancy and postpartum. The invention also discloses a method of detecting diabetes in a subject. In a typical embodiment, the method comprises measuring the amount of one, two, three or more metabolic markers present in a test sample obtained from the subject, and comparing the amount of the metabolic marker present in the test sample to a control sample. The method further comprises identifying a subject as susceptible to, or as having, diabetes if the amount of marker present in the test sample is increased or decreased relative to the control sample. According to the invention, the metabolic marker comprises 1-methylhistidine.

An amount of marker is considered increased or decreased if it differs by a statistically significant amount from the amount present in the control. In some embodiments, the difference is an increase or decrease of at least 10%; in other embodiments, the difference is at least 20%, 30%, 40%, 50% or more. In other embodiments, a reference range has been identified for the amount of the metabolic marker present in a normal, control sample, and a test sample having an amount of the marker that is outside the reference range for that marker is susceptible to diabetes. The sample is a urine sample.

Metabolic markers to be measured are listed in Tables 1, 2, and 3. According to the invention, the metabolic marker comprises 1-methylhistidine. The metabolic markers can be selected from the group consisting of: adipate, methylsuccinate, pyroglutamine, cytidine, 1-methylhistidine, 2-hydroxyisobutyrate, cystathionine, sucrose and anserine. Additional markers useful for the methods of the invention include: tiglyl carnitine, xylonate, quinolinate, pro hydroxy pro, N-acetylthreonine, trigonelline, and alpha-CEHC glucuronide. Other groups of markers from which to select are described in the examples below, and/or are based on the gestational stage of the subject, the normalization used, and/or the method of analysis to be employed. For example, markers for use in a method of detecting diabetes or susceptibility to diabetes during the first trimester of pregnancy can be selected from the group consisting of: adipate, methylsuccinate, pyroglutamine, cytidine, 1-methylhistidine, 2-hydroxyisobutyrate, cystathionine, sucrose, glutamate, galactose, 3-hydroxy-3-methylglutarate, and anserine. Representative markers useful for the methods relating to pregnant subjects at 24-38 weeks gestation include: tiglyl carnitine, xylonate, quinolinate, pro hydroxy pro, N-acetylarginine, itaconate, leucine, scyllo-inositol, thymine, carnitine, acetylcarnitine, agmatine, sorbose, N-acetylthreonine, and trigonelline, Representative markers useful for the methods of the invention relating to subjects who are 4-12 weeks postpartum include: quinolinate, pro hydroxy pro, N-acetylarginine, trigonelline, lactose, 4-hydroxyphenylacetate, gamma-CEHC glucuronide, glutarate, dihydrobiopterin, sucrose, sarcosine, glycocholate, homoserine, pyrogluatime, 5-methylthioadenosine, nicotinate, and alpha-CEHC glucuronide. These postpartum markers can also serve as markers for nonpregnant subjects.

Examples of markers for which an increase relative to control is indicative of diabetes are listed in Example 3 below; and examples of markers for which a decrease relative to control is indicative of diabetes are also listed in Example 3 below. As noted in Example 3, the markers that increase or decrease with GDM relative to normal are different at 12 weeks gestation, 24 weeks gestation, and postpartum. As shown in the examples below, more than one marker may be used in combination to identify the presence of GDM.

### Table 1

| | |
|---|---|
| | 3-(3-hydroxyphenyl)proprionate |
| Acetylcarnitine | Itaconate |
| Adipate | Lactose |
| Agmatine | Leucine |
| Anserine | 1-methylhistidine |
| Carnitine | Methylsuccinate |
| Cystathionine | 5-methylthioadenosine |
| Cytidine | N-acetylarginine |
| Dihydrobiopterin | N-acetylthreonine |
| 3-fucosyllactose | Nicotinate |
| Galactose | Pro-hydroxy-pro |
| Gamma-CEHC glucuronide | Pyroglutamine |
| Gluconate | Quinolinate |
| Glutamate | Scyllo-inositol |
| Glutarate | Sorbose |
| Glycocholate | Sucrose |
| Homoserine | Thymine |
| 2-hydroxyisobutyrate | Tiglyl carnitine |
| 3-hydroxy-3-methylglutarate | Trigonelline |
| hydroxyphenylacetate | Xylonate |

### Table 2

| | |
|---|---|
| Alpha-CEHC glucuronide | Pro hydroxy pro |
| Anserine | 2-hydroxyisobutyrate |
| Methylsuccinate | N-acetylthreonine |
| Xylonate | Carnitine |
| Tigyl carnitine | Trigonelline |
| Quinolinate | |

### Table 3

3-fucosyllactose
3-(3-hydroxyphenyl)proprionate
Itaconate
1-methylhistidine
Quinolinate
Sucrose

In one embodiment, the measuring comprises chromatography or spectrometry. The chromatography can be gas or liquid chromatography. The spectrometry can be mass spectrometry. Other known methods of metabolic marker detection are also contemplated, and may be selected based on the characteristics of the individual marker of interest. Examples of other assays that can be employed include immunoassay and electrochemical detection. Measures of test samples can be compared directly to controls, such as comparing the concentration of the metabolic marker present in the test sample to the concentration of the metabolic marker in a control sample or to a known normal concentration of the metabolic marker. The metabolite concentration is normalized with respect to a selected normalizing marker, such as creatinine or osmolality.

In one embodiment, the subject is pregnant, and the screening is for gestational diabetes mellitus (GDM). The method is typically performed when subject is at least 6 weeks pregnant. The method is performed when the subject is 6-17 weeks pregnant, typically when the subject is 6-14 weeks pregnant.

Testing can also be performed later in pregnancy, such as at about 22-30 weeks gestation, or at term. Testing can be performed for patients contemplating pregnancy. It can also be used in nonpregnant populations as a general method of screening for diabetes and susceptibility to diabetes, such as type 2 diabetes mellitus.

Kits

For use in the diagnostic applications described herein, kits are also disclosed. Such kits can comprise a carrier, package or container that is compartmentalized to receive one or more containers such as vials, tubes, and the like, each of the container(s) comprising one of the separate elements to be used in the method. The probes, antibodies and other reagents of the kit may be provided in any suitable form, including frozen, lyophilized, or in a pharmaceutically acceptable buffer such as TBS or PBS. The kit may also include other reagents required for utilization of the reagents in vitro or in vivo such as buffers (i.e., TBS, PBS), blocking agents (solutions including nonfat dry milk, normal sera, Tween-20 Detergent, BSA, or casein), and / or detection reagents (i.e., goat anti-mouse IgG biotin, streptavidin-HRP conjugates, allophycocyanin, B-phycoerythrin, R- phycoerythrin, peroxidase, fluors (i.e., DyLight, Cy3, Cy5, FITC, HiLyte Fluor 555, HiLyte Fluor 647), and / or staining kits (i.e., ABC Staining Kit, Pierce)). The kits may also include other reagents and / or instructions for using antibodies, probes, and other reagents in commonly utilized assays described above such as, for example, liquid or gas chromatography, spectrometry, electrochemical assay, flow cytometric analysis, ELISA, immunoblotting (i.e., western blot), immunocytochemistry, immunohistochemistry.

The kit can provide the reagent in purified form. The reagents can be immunoreagents that are provided in biotinylated form either alone or along with an avidin-conjugated detection reagent (i.e., antibody). The kit can include a fluorescently labeled immunoreagent which may be used to directly detect antigen. Buffers and the like required for using any of these systems are well-known in the art and may be prepared by the end-user or provided as a component of the kit. The kit may also include a solid support containing positive- and negative-control protein and / or tissue samples. For example, kits for performing spotting or western blot-type assays may include control cell or tissue lysates for use in SDS-PAGE or nylon or other membranes containing pre-fixed control samples with additional space for experimental samples.

The kit will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific application, and can also indicate directions for use, such as those described above. Directions and or other information can also be included on an insert which is included with the kit.

The kit can comprise a set of assay reagents suitable for detecting and/or measuring alpha-CEHC glucuronide, anserine, methylsuccinate, xylonate, tigyl carnitine, quinolinate, pro hydroxy pro, 2-hydroxyisobutyrate, N-acetylthreonine, carnitine, and trigonelline, or any other combination of markers described herein.

EXAMPLES

The following examples are presented to illustrate the present invention and to assist one of ordinary skill in making and using the same. The examples are not intended in any way to otherwise limit the scope of the invention.

**Example 1: Metabolomic Screening for Gestational Diabetes**

This example demonstrates that urinary metabolite concentrations can be used to identify pregnant women who will develop gestational diabetes mellitus (GDM).

**Methods**

Urine was collected from pregnant women from 6-38 weeks of gestation. Gestational diabetes was determined by standard clinical screening at 24-28 weeks of gestation: 50-g 1-h oral glucose challenge test (OGCT) followed by a 100-g 3-h oral glucose challenge test (OGTT) for those with an OGCT plasma glucose concentration of ≥140 mg/dl. The results of the OGCT and OGTT separated the gravidas into two groups: normal gravidas (NG) and those with GDM. Metabolon (Durham, NC) analyzed the urine samples on gas chromatography/mass spectrometry and liquid chromatography/mass spectrometry platforms for 441 low-molecular weight metabolites. Metabolite concentrations and metabolite levels normalized to urinary creatinine or osmolality were analyzed. The top 37 metabolites by random forest analysis that were found to have the most highly significantly different levels in GDM compared to NG were the following:

|  | Creatinine Normalized | Osmolality Normalized |
|---|---|---|
|  | **6-17 weeks' gestation** | |
|  | Adipate | 2-hydroxyisobutyrate |
|  | Methylsuccinate | Galactose |
|  | Pyroglutamine | 1-methylhistidine |
|  | Cytidine | Sucrose |
|  | 1-methylhistidine | 3-hydroxy-3-methylglutarate |
|  | 2-hydroxyisobutyrate | Gluconate |
|  | Glutamate | Anserine |
|  | Cystathionine | Methylsuccinate |
|  | Anserine | |
|  | **24-38 weeks' gestation** | |
|  | N-acetylarginine | N-acetylthreonine |
|  | Xylonate | Scyllo-inositol |
|  | Itaconate | Thymine |
|  | Tiglyl carnitine | Carnitine |
|  | Leucine | Acetylcarnitine |
|  | Agmatine | Sorbose |
|  | Sorbose | |
|  | **4-12 weeks postpartum** | |
|  | Quinolinate | Trigonelline |
|  | Pro-hydroxy-pro | Glycocholate |
|  | Lactose | Homoserine |
|  | 4-hydroxyphenylacetate | Pyroglutamine |
|  | Gamma-CEHC glucuronide | Sucrose |
|  | Glutarate | 5-methylthioadenosine |
|  | N-acetylarginine | Alpha-CEHC glucuronide |
|  | Dihydrobiopterin | Nicotinate |
|  | Trigonelline | |
|  | Sucrose | |
|  | Sarcosine | |

Further statistical scrutiny of these 37 candidate metabolites to distinguish GDM from NG by simultaneous classification tree analysis revealed the following optimal sensitivity, accuracy, and ROC area under the curve (AUC) using 1-2 metabolites:

Creatinine normalization

| Weeks | GDM (n) | NG (n) | Sensitivity | Specificity | Accuracy | ROC AUC | Metabolites Used |
|---|---|---|---|---|---|---|---|
| 6-17 GA | 9 | 16 | 100% | 100% | 100% | 1.000 | anserine, methylsuccinate |
| 24-38 GA | 12 | 16 | 92% | 88% | 90% | 0.909 | xylonate tiglyl carnitine |
| 4-12 PP | 12 | 16 | 83% | 100% | 92% | 0.943 | quinolinate pro hydroxy pro |

Osmolality normalization

| Weeks | GDM (n) | NG (n) | Sensitivity | Specificity | Accuracy | ROC AUC | Metabolites Used |
|---|---|---|---|---|---|---|---|
| 6-17 | 9 | 16 | 78% | 94% | 86% | 0.858 | 2-hydroxyisobutyrate |

(continued)

| Weeks | GDM (n) | NG (n) | Sensitivity | Specificity | Accuracy | ROC AUC | Metabolites Used |
|-------|---------|--------|-------------|-------------|----------|---------|------------------|
| 24-38 | 12 | 16 | 92% | 94% | 93% | 0.948 | N-acetylthreonine carnitine |
| 4-12 PP | 12 | 16 | 100% | 94% | 97% | 0.969 | trigonelline α-CEHC-glucuronide |
| GA, gestational age; PP, postpartum | | | | | | | |

**Interpretation**

Eleven metabolites have been identified that, in various combinations, have a high degree of accuracy in identifying gravidas who will develop gestational diabetes, as diagnosed by current clinical practice. In this study, the urinary analysis had up to 100% sensitivity, specificity and accuracy in early pregnancy (6-17 weeks). The detection of GDM in early pregnancy (-0.25 term) enables the clinical management of these patients (e.g., counseling, life-style changes, and glucose monitoring) long before GDM is normally diagnosed by current screening methods at about 26-30 weeks of gestation (~0.75 term). Commencing GDM surveillance and management at 10-12 weeks of gestation will likely reduce maternal and fetal and maternal morbidity related to hyperglycemia of GDM.

Urinary metabolites were also useful in later gestation (~0.75 term) in that it had 92% sensitivity for identifying GDM. Thus, a positive urinary metabolite screen would potentially eliminate the need for further GDM testing.

In postpartum (nonpregnant) women, the urinary metabolite screen identified all women who had had GDM (sensitivity and specificity up to 100%). These results suggest that such urinary metabolite testing would be useful for women who are considering pregnancy. A positive preconception screen would enable appropriate counseling and dietary alterations before pregnancy.

The ability of urinary metabolites to identify postpartum women (~6 weeks after delivery) who had GDM suggests that urinary metabolite screening would also be useful in identifying those in the general population who are either disposed to or actually have type 2 diabetes mellitus.

Metabolomic analysis have been performed on urine in pregnant women. Urine has particular advantages over blood tests because it is readily available, collected without discomfort to the patient, and does not require expensive skilled personnel for collection. But other body fluids can also be sampled for metabolomic analysis:

a) *Blood.* Pregnant women routinely have blood tests as part of the first prenatal visit. Therefore, metabolomic analysis of plasma samples should be done to establish whether plasma levels of these 11 or more (up to 37 total) metabolites are useful for identifying those who will develop GDM. Nonpregnant subjects commonly have screening blood tests (e.g., complete blood count, hemoglobin A1C, glucose) as part of routine health care. Thus, metabolomic analysis of plasma levels of these 11 or more (up to 37 total) metabolites should be done to determine whether these blood metabolites are useful in identifying those who are either disposed to type 2 diabetes mellitus or have the disorder.

b) *Saliva.* Another body fluid that is easily sampled is saliva. Thus, metabolomic analysis of saliva should be performed to determine whether these 11 or more (up to 37 total) metabolites in saliva can predict gravidas who will develop GDM as well as nonpregnant subjects disposed to type 2 diabetes mellitus.

**Example 2: Statistical Analysis of Metabolites Associated With Gestational Diabetes**

In this example, the metabolites measured at ~12 weeks (6-17 weeks gestation), ~27 weeks (24-32 weeks gestation) and ~6 weeks post partum were explored using both creatinine normalized and osmolality normalized data. The results show that the optimal metabolites for separating GDM from normals in this study differ by group. Thus, for each week and normalization method, a different subset of 8 to 11 metabolites from the 441 metabolites were used based on random forest analysis. There are 6 analyses (3 collection times with 2 normalization methods) using up to 11 metabolites in any one analysis.

**Statistical methods**

The statistical methods are the same as described in Example 1 above.

<u>Univariate</u> - A nonparametric ROC was carried out on each metabolite separately to identify the best threshold, sensitivity, specificity and unweighted accuracy for each. Unweighted accuracy is defined as

$$\text{Unweighted accuracy} = (\text{sensitivity} + \text{specificity})/2$$

Means and standard deviations by group are reported on the log (base e) scale since the distribution of the metabolites is much closer to a normal (Gaussian) distribution on the log scale. Thus the original scale GDM/normal mean ratio is computed as the antilog of the log scale mean differences and is also the ratio of geometric means, NOT the ratio of arithmetic means. The geometric mean is also the theoretical median when the log scale data follow a normal distribution.

$$(\log (GDM/normal)) = \log(GDM) - \log(normal), \ GDM/normal=\exp(\log(GDM) - \log(normal))$$

To be conservative, p values were computed using the nonparametric Wilcoxon rank sum test.

Multivariate - The nonparametric classification tree model was used to simultaneously evaluate all 8 metabolites and the corresponding sensitivity, specificity and accuracy is reported.

**Results:**

Univariate - The tables below show the univariate log scale means, standard deviations, thresholds, sensitivity, specificity and unweighted accuracy by normalization method and time (12 weeks, 24 weeks, post partum). The original scale GDM/normal geometric mean ratio is also reported. For example, at 12 weeks using creatinine normalization, methylsuccinate provides the highest unweighted nominal accuracy of 90.6% and has a GDM/normal mean ratio of 3.1 on the original scale. Using osmo normalized data at 12 weeks, 2 hydroxyisobutyrate provides the highest unweighted nominal accuracy of 85.8% and has a mean ratio of 1.47.

**Univariate metabolite comparison: GDM vs normal, log e scale**

nominal specificity (spec) and sensitivity (sens). P values from Wilcoxon rank sum test

\* original scale geometric mean ratio

**Creatinine normalized, 12 wks, 9 GDM, 16 normals**

| metabolite | Normal Mean | Normal SD | GDM Mean | GDM SD | p value | cutpt | spec | sens | accuracy | GDM/Normal mean ratio * | avg | GDM/Normal SD ratio |
|---|---|---|---|---|---|---|---|---|---|---|---|---|
| adipate | -0.092 | 0.56 | 0.088 | 0.242 | 0.0096 | 0.069 | 87.50% | 77.80% | 82.60% | 1.2 | -0.002 | 0.43 |
| methylsuccinate | -0.391 | 0.632 | 0.741 | 1.526 | 0.0149 | -0.179 | 81.30% | 100.00% | 90.60% | 3.1 | 0.175 | 2.41 |
| pyroglutamine | 0.095 | 0.374 | -0.137 | 0.307 | 0.0494 | -0.084 | 75.00% | 88.90% | 81.90% | 0.79 | -0.021 | 0.82 |
| cytidine | -0.101 | 0.882 | 0.372 | 0.511 | 0.0954 | -0.291 | 62.50% | 100.00% | 81.30% | 1.61 | 0.136 | 0.58 |
| X1.methylhistidine | -0.757 | 1.219 | 0.012 | 0.556 | 0.008 | -0.007 | 93.80% | 66.70% | 80.20% | 2.16 | -0.372 | 0.46 |
| X2.hydroxyisobutyrate | -0.229 | 0.333 | 0.153 | 0.319 | 0.008 | -0.259 | 62.50% | 100.00% | 81.30% | 1.47 | -0.038 | 0.96 |
| glutamate | -0.186 | 0.559 | 0.68 | 0.871 | 0.0196 | 0.556 | 93.80% | 66.70% | 80.20% | 2.38 | 0.247 | 1.56 |
| cystathionine | -1.086 | 0.886 | -0.16 | 1.133 | 0.0471 | -0.374 | 93.80% | 77.80% | 85.80% | 2.52 | -0.623 | 1.28 |
| anserine | -0.845 | 1.395 | 1.427 | 1.899 | 0.0084 | 2.031 | 100.00% | 55.60% | 77.80% | 9.7 | 0.291 | 1.36 |
| X1.methylxanthine | 0.542 | 1.579 | -0.495 | 1.02 | 0.1076 | -0.149 | 68.80% | 88.90% | 78.80% | 0.35 | 0.024 | 0.65 |

## Osmolality normalized, 12 wks, 9GDM, 16 normals

| metabolite | Normal Mean | Normal SD | GDM Mean | GDM SD | p value | cutpt | spec | sens | accuracy | GDM/Normal mean ratio * | avg | GDM/Normal SD ratio |
|---|---|---|---|---|---|---|---|---|---|---|---|---|
| X2.hydroxyisobutyrate | -0.141 | 0.39 | 0.245 | 0.256 | 0.0043 | 0.19 | 93.80% | 77.80% | 85.80% | 1.47 | 0.052 | 0.66 |
| galactose | 0.18 | 1.139 | -0.805 | 0.817 | 0.0203 | -0.222 | 68.80% | 77.80% | 73.30% | 0.37 | -0.312 | 0.72 |
| X1.methylhistidine | -0.722 | 1.244 | 0.077 | 0.386 | 0.0231 | -0.205 | 75.00% | 88.90% | 81.90% | 2.22 | -0.322 | 0.31 |
| X5.acetylamino.6.amino.3.methyluracil | 0.325 | 1.05 | -0.674 | 0.677 | 0.0139 | 0.361 | 56.30% | 88.90% | 72.60% | 0.37 | -0.175 | 0.64 |
| sucrose | 0.118 | 0.788 | -0.854 | 1.068 | 0.0165 | -0.505 | 81.30% | 66.70% | 74.00% | 0.38 | -0.368 | 1.35 |
| X3.hydroxy.3.methylglutarate | -0.152 | 0.537 | 0.137 | 0.286 | 0.0369 | -0.143 | 62.50% | 100.00% | 81.30% | 1.34 | -0.008 | 0.53 |
| gluconate | -0.136 | 0.415 | 0.259 | 0.323 | 0.0165 | 0.091 | 81.30% | 77.80% | 79.50% | 1.48 | 0.062 | 0.78 |
| anserine | -0.868 | 1.266 | 1.385 | 1.785 | 0.005 | 2.079 | 100.00% | 55.60% | 77.80% | 9.51 | 0.258 | 1.41 |
| methylsuccinate | -0.402 | 0.807 | 0.707 | 1.596 | 0.0149 | 0.465 | 100.00% | 66.70% | 83.30% | 3.03 | 0.152 | 1.98 |

## Creatinine normalized, 24 wks, 12 GDM, 16 normals

| metabolite | Normal Mean | Normal SD | GDM Mean | GDM SD | p value | cutpt | spec | sens | accuracy | GDM/Normal mean ratio * | avg | GDM/Normal SD ratio |
|---|---|---|---|---|---|---|---|---|---|---|---|---|
| N.acetylarginine | -0.178 | 0.205 | 0.074 | 0.203 | 0.0031 | -0.046 | 87.50% | 75.00% | 81.30% | 1.29 | -0.052 | 0.99 |
| xylonate | 0.022 | 0.275 | 0.375 | 0.492 | 0.0113 | 0.223 | 93.80% | 66.70% | 80.20% | 1.42 | 0.198 | 1.79 |
| itaconate..methylene succinate. | 0.092 | 0.459 | 0.591 | 0.348 | 0.0022 | 0.533 | 93.80% | 66.70% | 80.20% | 1.65 | 0.342 | 0.76 |
| tiglyl.carnitine | -0.227 | 0.299 | 0.126 | 0.255 | 0.0018 | -0.226 | 62.50% | 91.70% | 77.10% | 1.42 | -0.051 | 0.85 |
| leucine | 0.153 | 0.702 | 0.444 | 0.387 | 0.0473 | 0.305 | 81.30% | 75.00% | 78.10% | 1.34 | 0.298 | 0.55 |
| agmatine | 0.542 | 0.38 | 0.168 | 0.566 | 0.0331 | 0.296 | 75.00% | 66.70% | 70.80% | 0.69 | 0.355 | 1.49 |
| sorbose | -0.243 | 1.619 | 0.325 | 0.733 | 0.2547 | -1.03 | 50.00% | 100.00% | 75.00% | 1.76 | 0.041 | 0.45 |
| abscisate | -0.907 | 0.672 | -0.244 | 0.853 | 0.0125 | -1.013 | 87.50% | 83.30% | 85.40% | 1.94 | -0.576 | 1.27 |

## Osmolality normalized, 24 wks, 12 GDM, 16 normals

| metabolite | Normal Mean | Normal SD | GDM Mean | GDM SD | p value | cutpt | spec | sens | accuracy | GDM/Normal mean ratio * | avg | GDM/Normal SD ratio |
|---|---|---|---|---|---|---|---|---|---|---|---|---|
| N.acetylthreonine | 0.326 | 0.175 | 0.035 | 0.234 | 0.001 | 0.052 | 93.80% | 58.30% | 76.00% | 0.75 | 0.181 | 1.33 |
| scyllo.inositol | 0.346 | 0.532 | -0.143 | 0.316 | 0.0037 | 0.178 | 68.80% | 91.70% | 80.20% | 0.61 | 0.102 | 0.6 |
| X3.7.dimethylurate | -0.388 | 0.864 | -1.217 | 0.557 | 0.0073 | -0.174 | 50.00% | 91.70% | 70.80% | 0.44 | -0.803 | 0.65 |
| thymine | 0.479 | 0.441 | 0.094 | 0.327 | 0.0257 | 0.403 | 62.50% | 83.30% | 72.90% | 0.68 | 0.286 | 0.74 |
| carnitine | 0.282 | 0.679 | -0.349 | 0.733 | 0.0331 | -0.349 | 87.50% | 50.00% | 68.80% | 0.53 | -0.034 | 1.08 |
| acetylcarnitine | 0.365 | 0.492 | -0.111 | 0.644 | 0.0735 | -0.164 | 87.50% | 50.00% | 68.80% | 0.62 | 0.127 | 1.31 |
| sorbose | -0.264 | 1.723 | 0.215 | 0.807 | 0.3176 | -0.936 | 50.00% | 100.00% | 75.00% | 1.61 | -0.025 | 0.47 |
| abscisate | -1.005 | 0.721 | -0.428 | 0.864 | 0.0144 | -1.144 | 87.50% | 83.30% | 85.40% | 1.78 | -0.716 | 1.2 |

## Creatinine normalized, post partum, 12 GDM, 16 normals

| metabolite | Normal Mean | Normal SD | GDM Mean | GDM SD | p value | cutpt | spec | sens | accuracy | GDM/Normal mean ratio * | avg | GDM/Normal SD ratio |
|---|---|---|---|---|---|---|---|---|---|---|---|---|
| quinolinate | -0.266 | 0.244 | 0.18 | 0.242 | 0.0001 | -0.11 | 81.30% | 91.70% | 86.50% | 1.56 | -0.043 | 0.99 |
| pro.hydroxy.pro | -0.254 | 0.763 | 0.342 | 0.464 | 0.015 | -0.085 | 75.00% | 91.70% | 83.30% | 1.82 | 0.044 | 0.61 |
| lactose | 1.363 | 1.064 | 2.21 | 0.565 | 0.0226 | 1.433 | 62.50% | 100.00% | 81.30% | 2.33 | 1.787 | 0.53 |
| X4.hydroxyphenylacetate | -0.072 | 0.377 | 0.323 | 0.347 | 0.013 | -0.013 | 68.80% | 91.70% | 80.20% | 1.48 | 0.126 | 0.92 |
| gamma.CEHC.glucuronide. | -0.382 | 0.683 | 0.401 | 0.492 | 0.0026 | 0 | 75.00% | 83.30% | 79.20% | 2.19 | 0.01 | 0.72 |
| glutarate..pentanedioate. | -0.363 | 0.501 | 0.081 | 0.576 | 0.0179 | -0.286 | 75.00% | 100.00% | 87.50% | 1.56 | -0.141 | 1.15 |
| N.acetylarginine | 0.022 | 0.436 | 0.23 | 0.224 | 0.0327 | 0.023 | 68.80% | 91.70% | 80.20% | 1.23 | 0.126 | 0.51 |
| dihydrobiopterin | -0.432 | 0.728 | 0.187 | 0.292 | 0.0114 | -0.123 | 62.50% | 91.70% | 77.10% | 1.86 | -0.123 | 0.4 |
| trigonelline..N..methyl nicotinate. | 0.204 | 0.534 | -0.398 | 0.578 | 0.0083 | 0.057 | 62.50% | 75.00% | 68.80% | 0.55 | -0.097 | 1.08 |
| sucrose | 0.394 | 0.713 | -0.029 | 0.872 | 0.0331 | -0.07 | 87.50% | 66.70% | 77.10% | 0.66 | 0.182 | 0.82 |
| sarcosine..N.Methylglycine. | -0.45 | 0.526 | 0.238 | 0.513 | 0.0043 | -0.163 | 68.80% | 83.30% | 76.00% | 1.99 | -0.106 | 1.03 |

**Osmolality normalized, post partum, 12 GDM, 16 normals**

| metabolite | Normal Mean | Normal SD | GDM Mean | GDM SD | p value | cutpt | spec | sens | accuracy | GDM/Normal mean ratio * | avg | GDM/Normal SD ratio |
|---|---|---|---|---|---|---|---|---|---|---|---|---|
| trigonelline..N..methyl nicotinate. | 0.234 | 0.603 | -0.574 | 0.501 | 0.0003 | 0.101 | 75.00% | 100.00% | 87.50% | 0.45 | -0.17 | 0.83 |
| glycocholate | -0.305 | 0.66 | -0.877 | 0.767 | 0.024 | -1.011 | 81.30% | 66.70% | 74.00% | 0.56 | -0.591 | 1.16 |
| homoserine | -0.151 | 0.766 | -0.616 | 0.424 | 0.0168 | -0.432 | 75.00% | 75.00% | 75.00% | 0.63 | -0.384 | 0.55 |
| X4.hydroxyhippurate | 0.306 | 0.489 | -0.169 | 0.553 | 0.015 | -0.142 | 81.30% | 66.70% | 74.00% | 0.62 | 0.069 | 1.13 |
| pyroglutamine | 0.223 | 0.441 | -0.217 | 0.249 | 0.0083 | 0.05 | 50.00% | 91.70% | 70.80% | 0.64 | 0.003 | 0.57 |
| sucrose | 0.404 | 0.683 | -0.224 | 0.887 | 0.0172 | 0.013 | 75.00% | 66.70% | 70.80% | 0.53 | 0.09 | 1.3 |
| X5.methylthioadenosine..MTA. | -0.113 | 0.526 | -0.472 | 0.321 | 0.0373 | -0.178 | 68.80% | 83.30% | 76.00% | 0.7 | -0.292 | 0.61 |
| alpha.CEHC.glucuronide | -0.358 | 0.896 | 0.263 | 0.717 | 0.0421 | -0.529 | 50.00% | 100.00% | 75.00% | 1.86 | -0.048 | 0.8 |
| nicotinate | 0.03 | 0.834 | -0.315 | 0.433 | 0.053 | 0.006 | 62.50% | 91.70% | 77.10% | 0.71 | -0.143 | 0.52 |

Multivariate tree - The multivariate tree results were analyzed and reviewed by normalization method and time. The results including sensitivity, specificity and unweighted accuracy are summarized in the tables below.

At week 12, using the creatinine normalized data, the tree uses only anserine and methylsuccinate and creates four classification groups (terminal tree nodes). The nominal accuracy is 100%. Using the osmo-normalized data at week 12, the tree only uses 2-hydroxyisobutyrate (same as in the univariate results) and creates two classification groups. This tree correctly classifies 15 of 16 normals (94%) and 7 of 9 GDM (78%) for a nominal unweighted accuracy of (94% + 78%)/2 = 86%.

**Creatine normalization – with xenobiotics**

| week | GDM n | normal n | num candidate metabolites | nominal sensitivity | nominal specificity | nominal accuracy | ROC AUC | tree levels * | metabolites used |
|---|---|---|---|---|---|---|---|---|---|
| 12 | 9 | 16 | 11 | 100% | 100% | 100% | 1.00 | 2 | 4-vinylphenol sulfate, anserine |
| 24 | 12 | 16 | 9 | 100% | 94% | 97% | 0.99 | 3 | theobromine, agmatine |
| post partum | 12 | 16 | 14 | 92% | 100% | 96% | 0.95 | 3 | hydroxy-pro, dihydrobiopterin |

## Osmo normalization – with xenobiotics

| week | GDM n | normal n | num candidate metabolites | nominal sensitivity | nominal specificity | nominal accuracy | ROC AUC | tree levels* | metabolites used |
|---|---|---|---|---|---|---|---|---|---|
| 12 | 9 | 16 | 10 | 100% | 94% | 97% | 0.96 | 2 | 2-hydroxyisobutyrate, anserine |
| 24 | 12 | 16 | 12 | 100% | 75% | 88% | 0.96 | 2 | N-acetylthreonine, 1-3-7-trimethylurate |
| post partum | 12 | 16 | 12 | 100% | 100% | 100% | 1.00 | 3 | methylnicotinate, 3-hydroxyindolin-2-one, glycocholate |

* not including root level

## Best metabolites in Wulff report – 12 – with xenobiotics

| week | GDM n | normal n | num candidate metabolites | nominal sensitivity | nominal specificity | nominal accuracy | ROC AUC | tree levels* | metabolites used |
|---|---|---|---|---|---|---|---|---|---|
| creatinine | | | ?? | 89% | 69% | 79% | 0.736 | | |
| osmo | | | ?? | 100% | 100% | 100% | 1.000 | | |

## Creatine normalization – without xenobiotics

| week | GDM n | normal n | sensitivity | specificity | accuracy | ROC AUC | tree levels* | metabolites used |
|---|---|---|---|---|---|---|---|---|
| 12 | 9 | 16 | 100% | 100% | 100% | 1.000 | 3 | anserine, methyl succinate |
| 24 | 12 | 16 | 92% | 88% | 90% | 0.909 | 2 | xylonate, tiglyl carnitine |
| post partum | 12 | 16 | 83% | 100% | 92% | 0.943 | 2 | quinolinate, pro hydroxy pro |

## Osmolality normalization – without xenobiotics

| week | GDM n | normal n | sensitivity | specificity | accuracy | ROC AUC | tree levels* | metabolites used |
|---|---|---|---|---|---|---|---|---|
| 12 | 9 | 16 | 100% | 88% | 94% | 0.972 | 2 | anserine, sucrose |
| 24 | 12 | 16 | 92% | 94% | 93% | 0.948 | 2 | N acetylthreonine, carnitine |
| post partum | 12 | 16 | 100% | 94% | 97% | 0.969 | 2 | trigonelline N methylnicotinate, alpha CEHC glucuronide |

* not including root level

## Best metabolites in Wulff report – 12 – without xenobiotics

| week | GDM n | normal n | sensitivity | specificity | accuracy | ROC AUC | tree levels* | metabolites used |
|---|---|---|---|---|---|---|---|---|
| creatinine | | | 89% | 69% | 79% | 0.736 | | adipate, cytidine |
| osmo | | | 100% | 100% | 100% | 1.000 | | methylsuccinate,cytidine, methylhistidine,anserine |

## Example 3: Notable Metabolites by Random Forest Analysis

This example provides a list of metabolite markers of interest, as determined from the random forest analysis. The arrows indicate whether the levels were higher or lower than in normals. This listing is followed by a series of tables that provide the tree with cutoffs and key metabolites.

### 6-17 Weeks Pregnant

| Osmolality Normalized | GDM/Normal Mean Ratio |
|---|---|
| 2-hydroxyisobutyrate | ↑ |
| Galactose | ↓ |
| 1-methylhistidine | ↑ |
| Sucrose | ↓ |
| 3-hydroxy-3-methylglutarate | ↑ |
| Gluconate | ↑ |
| Anserine | ↑ |
| Methylsuccinate | ↑ |

*Creatinine Normalized*

| | |
|---|---|
| Adipate | ↑ |
| Methylsuccinate | ↑ |
| Pyroglutamine | ↓ |
| Cytidine | ↑ |
| 1-methylhistidine | ↑ |
| 2-hydroxyisobutyrate | ↑ |
| Glutamate | ↑ |
| Cystathionine | ↑ |
| Anserine | ↑ |

**24-38 Weeks Pregnant**

| *Osmolality Normalized* | *GDM/Normal Mean Ratio* |
|---|---|
| N-acetylthreonine | ↓ |
| Scyllo-inositol | ↓ |
| Thymine | ↓ |
| Carnitine | ↓ |
| Acetylcarnitine | ↓ |
| Sorbose | ↑ |

*Creatinine Normalized*

| | |
|---|---|
| N-acetylarginine | ↑ |
| Xylonate | ↑ |
| Itaconate | ↑ |
| Tiglyl carnitine | ↑ |
| Leucine | ↑ |
| Agmatine | ↓ |
| Sorbose | ↑ |

**POSTPARTUM**

| *Osmolality Normalized* | *GDM/Normal Mean Ratio* |
|---|---|
| Trigonelline | ↓ |
| Glycocholate | ↓ |
| Homoserine | ↓ |
| Pyroglutamine | ↓ |
| Sucrose | ↓ |
| 5-methylthioadenosine | ↓ |
| Alpha-CEHC glucuronide | ↑ |
| Nicotinate | ↓ |

*Creatinine Normalized*

| | |
|---|---|
| Quinolinate | ↑ |
| Pro-hydroxy-pro | ↑ |

(continued)

| *Creatinine Normalized* | |
|---|---|
| Lactose | ↑ |
| 4-hydroxyphenylacetate | ↑ |
| Gamma-CEHC glucuronide | ↑ |
| Glutarate | ↑ |
| N-acetylarginine | ↑ |
| Dihydrobiopterin | ↑ |
| Trigonelline | ↓ |
| Sucrose | ↓ |
| Sarcosine | ↑ |

## Cross Tables From Tree Analyses

| creatinine normalization, 12 weeks | | | |
|---|---|---|---|
| | anserine < 0.405 | 0.405 <= anserine<14.32 | 14.32 <=anserine |
| methylsuccinate >=0.9294 | Normal | **GDM** | **GDM** |
| methylsuccinate <0.9294 | Normal | normal | **GDM** |

Anserine is higher in GDM compared to normals. Methylsuccinate is higher in GDM compared to normals in those with anserine between 0.405 and 14.32.

| creatinine normalization, 24 weeks | | |
|---|---|---|
| | Xylonate < 1.2739 | 1.2739 <=Xylonite |
| Tiglyl carnitine >= 1.2849 | **GDM** | **GDM** |
| Tiglyl carnitine < 1.2849 | Normal | **GDM** |

Both xylonite and tiglyl carnitine are higher in GDM compared to normal. Normals are lower on both Xylonate and tigly carnitine.

| creatinine normalization, post partum | | |
|---|---|---|
| | Quinolinate < 0.9201 | 0.9201 <=Quinolinate |
| Pro hyrdoxy pro >= 1.0385 | Normal | **GDM** |
| Pro hyrdoxy pro < 1.0385 | Normal | normal |

If Quinolinate >= 0.9202, pro hydroxyl pro is higher in GDM than normals. If pro hydoxy pro >=1.0385, quinolinate is higher in GDM than normals. Those with GDM are higher on both Quinolinate and pro hydroxy pro.

| osmo normalization, 12 weeks | |
|---|---|
| 2. hydroxyisobutyrate < 1.2093 | 1.2092 <=2. hydroxyisobutyrate |
| normal | **GDM** |

2. hydroxyisobutyrate is higher in GDM compared to normals.

osmo normalization, 24 week s

|  | N.acetylthreonine <1.0802 | 1.0802 <=N.acetylthreonine |
|---|---|---|
| Carnitine >=0.8113 | **GDM** | normal |
| Carnitine < 0.8113 | **GDM** | **GDM** |

N.acethythreonine is lower in GDM compared to normals in those with Carnitine >=0.8113.

Normals are higher on both N.acethythreonine and Carnitine.

osmo normalization, post partum

|  | trig < 1.1356 | 1.1356 <= trig |
|---|---|---|
| Alpha CEHC >= 0.6393 | **GDM** | normal |
| Alpha CEHC < 0.6393 | Normal | normal |
| trig = trigonelline N methylnicotinate<br>alpha CEHC = alpha CEHC glucuronide | | |

Those with GDM have both lower trig and higher alpha CEHC compared to normals.

### Example 4: Metabolic Signature of Gestational Diabetes in First Trimester

This example describes the distinctive metabolic profile that can be observed in early pregnancy for gravidas who develop GDM.

Study Design: Urine samples were collected at <14 weeks' gestation from 370 healthy gravidas with singletons. Abnormal OGCTs (24-28 wks) were followed by 100-g OGTT. Nine GDM subjects were matched with 16 normal gravidas (NG) . Urine samples were analyzed on GC/MS and LC/MS/MS platforms by Metabolon for 441 low molecular weight metabolites. Scaled biochemical levels were normalized separately to creatinine or osmolality. Random forests (RF) analysis identified 8 biochemicals that differed most significantly between GDM and NG. A nonparametric ROC identified the optimal threshold, sensitivity and specificity for each metabolite in predicting GDM. A non-parametric classification tree model (CART) was used to evaluate simultaneously the 8 metabolites and compute the corresponding sensitivity and specificity. P-values were calculated by unpaired t-test and Fisher's exact test where appropriate. Results are expressed as means $\pm$ SE.

Results: NG and GDM had similar age (NG: 33 $\pm$ 1.2; GDM: 34 $\pm$ 1.1 years), BMI (NG: 25.6 $\pm$ 1.4; GDM: 28.4 $\pm$ 2.3 kg/m2), ethnicity, parity, and GA at urine collection (NG:8.8 $\pm$ 0.7; GDM: 10.1 $\pm$ 0.9 weeks). CART analysis for the top 8 creatinine-normalized metabolites that differed between NG and GDM showed that 100% sensitivity and specificity (no misclassification) was obtained using a decision rule based on anserine and methylsuccinate. CART analysis of these same metabolites referenced to osmolality revealed a 100% sensitivity and 88% specificity (2 normals misclassified) using a decision rule based on anserine and sucrose.

Conclusion: RF and CART were surprisingly accurate in identifying critical metabolites that separate NG and GDM in early pregnancy.

### Example 5: Metabolic Signature of Gestational Diabetes Throughout Pregnancy

This example describes the distinctive metabolic profile that can be observed in different stages of pregnancy for gravidas who develop GDM.

Methods: Fed-state urine samples were collected at 6-14 weeks' and 22-32 weeks' gestation and 6 weeks postpartum from 375 healthy gravidas with singletons. Abnormal 50-g glucose challenge tests (GCT) at 24-28 weeks' gestation were followed by 100-g GTT. Nine GDM subjects were matched with 16 normal gravidas (NG). Urine samples were analyzed on GC/MS and LC/MS/MS platforms for 441 metabolites (Metabolon). Scaled metabolite levels were normalized to creatinine. Random forest analysis identified 8 biochemicals that most distinguished GDM from NG. ROC analysis identified optimal threshold, sensitivity, and specificity of each metabolite for predicting GDM. Classification tree model (CART) was used to compute simultaneously the corresponding sensitivity and specificity for each metabolite. P-values were calculated by unpaired t-test and Fisher's exact test where appropriate.

Results: GDM and NG were similar in age, ethnicity, parity, and BMI. Results of CART analysis of the 8 most discriminating metabolites for predicting GDM are shown in the following table.

| Time | Metabolites | Sensitivity (%) | Specificity (%) | Accuracy (%) |
|---|---|---|---|---|
| 6-14 weeks | 1-methylhistidine | 89 | 100 | 94 |
| | sucrose | | | |
| 22-32 weeks | Itaconate | 92 | 94 | 93 |
| | 3-fucosyllactose | | | |
| Postpartum | quinolinate | 92 | 100 | 96 |
| | 3-(3-hydroxyphenyl)proprion ate | | | |

Conclusions: The most discriminating biochemicals in tree analysis differ according to gestational age with a similar high accuracy in predicting GDM regardless of the time of urine collection.

From the foregoing it will be appreciated that, although specific embodiments of the invention have been described herein for purposes of illustration, various modifications may be made without deviating from the scope of the invention. Accordingly, the invention is not limited except as by the appended claims.

**Claims**

1. A method of screening for susceptibility to, or detecting, gestational diabetes mellitus in a subject, wherein the subject is 6-17 weeks pregnant, the method comprising:

    measuring the amount of a metabolic marker present in a test sample of urine obtained from the subject;
    comparing the amount of the metabolic marker present in the test sample to a control sample;
    identifying a subject as susceptible to, or having, diabetes if the amount of marker present in the test sample is increased or decreased relative to the control sample;
    wherein the metabolic marker comprises 1-methylhistidine;
    wherein the, or each, metabolic marker concentration is normalized with respect to a selected normalizing marker selected from creatinine or osmolality.

2. The method of claim 1 of screening for susceptibility to gestational diabetes mellitus in a subject, wherein the subject is 6-17 weeks pregnant, the method comprising:

    measuring the amount of a metabolic marker present in a test sample of urine obtained from the subject;
    comparing the amount of the metabolic marker present in the test sample to a control sample;
    identifying a subject as susceptible to diabetes if the amount of marker present in the test sample is increased or decreased relative to the control sample;
    wherein the metabolic marker comprises 1-methylhistidine;
    wherein the, or each, metabolic marker concentration is normalized with respect to a selected normalizing marker selected from creatinine or osmolality.

3. The method of claim 2, wherein the measuring comprises chromatography or spectrometry;
    wherein, optionally, the chromatography is gas or liquid chromatography; or
    wherein, optionally, the spectrometry is mass spectrometry.

4. The method of claim 2, wherein the subject is 6-14 weeks pregnant.

5. The method of claim 2, wherein the metabolic marker is normalized to urinary creatinine or osmolality.

6. The method of claim 1 of screening for susceptibility to gestational diabetes mellitus in a subject, wherein the subject is 6-17 weeks pregnant, the method comprising:

    measuring the amount of at least two metabolic markers present in a test sample of urine obtained from the subject;
    comparing the amount of the metabolic marker present in the test sample to a control sample;

identifying a subject as susceptible to diabetes if the amount of the markers present in the test sample is increased or decreased relative to the control sample;

wherein the metabolic markers comprise 1-methylhistidine and at least one marker selected from the group consisting of: alpha-CEHC glucuronide, anserine, methylsuccinate, xylonate, tigyl carnitine, quinolinate, pro hydroxy pro, 2-hydroxyisobutyrate, N-acetylthreonine, carnitine, and trigonelline;

wherein the, or each, metabolic marker concentration is normalized with respect to a selected normalizing marker selected from creatinine or osmolality.

7. The method of claim 6, wherein at least three markers are measured.

8. The method of any of claims 1 to 5 or 7 wherein the marker comprises 1-methylhistidine and any combination of one or more of the markers selected from the group consisting of: alpha-CEHC glucuronide, anserine, methylsuccinate, xylonate, tigyl carnitine, quinolinate, pro hydroxy pro, 2-hydroxyisobutyrate, N-acetylthreonine, carnitine, trigonelline, 3-fucosyllactose, 3-(3-hydroxyphenyl)proprionate, itaconate, and sucrose.

9. The method of any of claims 1 to 5 or 7 wherein the combination of markers are:

Sucrose and 1-methylhistidine;
Methylsuccinate, anserine and 1-methylhistidine;
Methylsuccinate, sucrose and 1-methylhistidine;
Sucrose, anserine and 1-methylhistidine; and
Methylsuccinate, sucrose, anserine, and 1-methylhistidine.

10. The method of any of claims 1 to 5 or 7 wherein the combination of markers are: adipate, methylsuccinate, pyroglutamine, cytidine, 1-methylhistidine, 2-hydroxyisobutyrate, cystathionine, sucrose and anserine.

11. The method of any of claims 1 to 5 or 7 wherein the combination of markers are: adipate, methylsuccinate, pyroglutamine, cytidine, 1-methylhistidine, 2-hydroxyisobutyrate, cystathionine, sucrose, glutamate, galactose, 3-hydroxy-3-methylglutarate, and anserine.

**Patentansprüche**

1. Verfahren zum Screening für die Anfälligkeit für oder zum Nachweis von Gestationsdiabetes mellitus bei einem Subjekt, wobei das Subjekt 6-17 Wochen schwanger ist, wobei das Verfahren Folgendes umfasst:

Messen der Menge eines metabolischen Markers, der in einer Untersuchungsprobe von Urin vorliegt, der von dem Subjekt erhalten wurde;
Vergleichen der Menge des metabolischen Markers, der in der Untersuchungsprobe vorliegt, mit einer Kontrollprobe;
Identifizieren eines Subjekts als für Diabetes anfällig oder diesen aufweisend, wenn die Menge des Markers, der in der Untersuchungsprobe vorliegt, bezüglich der Kontrollprobe erhöht oder erniedrigt ist;
wobei der metabolische Marker 1-Methylhistidin umfasst;
wobei die oder jede Konzentration des metabolischen Markers in Bezug auf einen ausgewählten Normalisierungsmarker, der aus Kreatinin oder Osmolalität ausgewählt ist, normalisiert wird.

2. Verfahren nach Anspruch 1 zum Screening für die Anfälligkeit für Gestationsdiabetes mellitus bei einem Subjekt, wobei das Subjekt 6-17 Wochen schwanger ist, wobei das Verfahren Folgendes umfasst:

Messen der Menge eines metabolischen Markers, der in einer Untersuchungsprobe von Urin vorliegt, der von dem Subjekt erhalten wurde;
Vergleichen der Menge des metabolischen Markers, der in der Untersuchungsprobe vorliegt, mit einer Kontrollprobe;
Identifizieren eines Subjekts als für Diabetes anfällig, wenn die Menge des Markers, der in der Untersuchungsprobe vorliegt, bezüglich der Kontrollprobe erhöht oder erniedrigt ist;
wobei der metabolische Marker 1-Methylhistidin umfasst;
wobei die oder jede Konzentration des metabolischen Markers in Bezug auf einen ausgewählten Normalisierungsmarker, der aus Kreatinin oder Osmolalität ausgewählt ist, normalisiert wird.

**3.** Verfahren nach Anspruch 2, wobei das Messen Chromatographie oder Spektrometrie umfasst; wobei optional die Chromatographie Gas- oder Flüssigkeitschromatographie ist; oder wobei optional die Spektrometrie Massenspektrometrie ist.

**4.** Verfahren nach Anspruch 2, wobei das Subjekt 6-14 Wochen schwanger ist.

**5.** Verfahren nach Anspruch 2, wobei der metabolische Marker auf Urin-Kreatinin oder -Osmolalität normalisiert wird.

**6.** Verfahren nach Anspruch 1 zum Screening für die Anfälligkeit für Gestationsdiabetes mellitus bei einem Subjekt, wobei das Subjekt 6-17 Wochen schwanger ist, wobei das Verfahren Folgendes umfasst:

Messen der Menge von mindestens zwei metabolischen Markern, die in einer Untersuchungsprobe von Urin vorliegen, der von dem Subjekt erhalten wurde;
Vergleichen der Menge des metabolischen Markers, der in der Untersuchungsprobe vorliegt, mit einer Kontrollprobe;
Identifizieren eines Subjekts als für Diabetes anfällig, wenn die Menge der Marker, die in der Untersuchungsprobe vorliegen, bezüglich der Kontrollprobe erhöht oder erniedrigt ist;
wobei die metabolischen Marker 1-Methylhistidin und mindestens einen Marker umfassen, der aus der Gruppe ausgewählt ist, die aus Folgenden besteht: Alpha-CEHC-Glucuronid, Anserin, Methylsuccinat, Xylonat, Tiglylcarnitin, Chinolinat, Pro-Hydroxy-Pro, 2-Hydroxyisobutyrat, N-Acetylthreonin, Carnitin und Trigonellin;
wobei die oder jede Konzentration des metabolischen Markers in Bezug auf einen ausgewählten Normalisierungsmarker, der aus Kreatinin oder Osmolalität ausgewählt ist, normalisiert wird.

**7.** Verfahren nach Anspruch 6, wobei mindestens drei Marker gemessen werden.

**8.** Verfahren nach einem der Ansprüche 1 bis 5 oder 7, wobei der Marker 1-Methylhistidin und jedwede Kombination von einem oder mehreren der Marker umfasst, die aus der Gruppe ausgewählt sind, die aus Folgenden besteht: Alpha-CEHC-Glucuronid, Anserin, Methylsuccinat, Xylonat, Tiglylcarnitin, Chinolinat, Pro-Hydroxy-Pro, 2-Hydroxyisobutyrat, N-Acetylthreonin, Carnitin, Trigonellin, 3-Fucosyllactose, 3-(3-Hydroxyphenyl)propionat, Itaconat und Saccharose.

**9.** Verfahren nach einem der Ansprüche 1 bis 5 oder 7, wobei die Kombination von Markern Folgende ist:

Saccharose und 1-Methylhistidin;
Methylsuccinat, Anserin und 1-Methylhistidin;
Methylsuccinat, Saccharose und 1-Methylhistidin;
Saccharose, Anserin und 1-Methylhistidin; und
Methylsuccinat, Saccharose, Anserin und 1-Methylhistidin.

**10.** Verfahren nach einem der Ansprüche 1 bis 5 oder 7, wobei die Kombination von Markern Folgende ist: Adipat, Methylsuccinat, Pyroglutamin, Cytidin, 1-Methylhistidin, 2-Hydroxyisobutyrat, Cystathionin, Saccharose und Anserin.

**11.** Verfahren nach einem der Ansprüche 1 bis 5 oder 7, wobei die Kombination von Markern Folgende ist: Adipat, Methylsuccinat, Pyroglutamin, Cytidin, 1-Methylhistidin, 2-Hydroxyisobutyrat, Cystathionin, Saccharose, Glutamat, Galactose, 3-Hydroxy-3-methylglutarat und Anserin.

**Revendications**

**1.** Méthode de criblage de la prédisposition à, ou de la détection, du diabète sucré gestationnel chez une patiente, la patiente étant enceinte de 6-17 semaines, la méthode comprenant :

la mesure de la quantité d'un marqueur métabolique présent dans un échantillon à tester d'urine obtenu à partir de la patiente ;
la comparaison de la quantité du marqueur métabolique présent dans l'échantillon à tester avec un échantillon témoin ;
l'identification d'une patiente comme étant prédisposée à, ou souffrant, d'un diabète, si la quantité de marqueur présent dans l'échantillon à tester est accrue ou réduite par rapport à l'échantillon témoin ;
dans laquelle le marqueur métabolique comprend de la 1-méthylhistidine ;
où la concentration du ou de chaque marqueur métabolique est normalisée par rapport à un marqueur sélectionné de normalisation, choisi parmi la créatinine ou l'osmolalité.

2. Méthode selon la revendication 1, de criblage de la prédisposition au diabète sucré gestationnel chez une patiente, la patiente étant enceinte de 6-17 semaines, la méthode comprenant :

la mesure de la quantité d'un marqueur métabolique présent dans un échantillon à tester d'urine obtenu à partir de la patiente ;
la comparaison de la quantité du marqueur métabolique présent dans l'échantillon à tester avec un échantillon témoin ;
l'identification d'une patiente comme étant prédisposée au diabète, si la quantité de marqueur présent dans l'échantillon à tester est accrue ou réduite par rapport à l'échantillon témoin ;
dans laquelle le marqueur métabolique comprend de la 1-méthylhistidine ;
où la concentration du ou de chaque marqueur métabolique est normalisée par rapport à un marqueur sélectionné de normalisation, choisi parmi la créatinine ou l'osmolalité.

3. Méthode selon la revendication 2, dans laquelle la mesure comprend une chromatographie ou une spectrométrie ; où, éventuellement, la chromatographie est une chromatographie gazeuse ou liquide ; ou où, éventuellement, la spectrométrie est une spectrométrie de masse.

4. Méthode selon la revendication 2, dans laquelle la patiente est enceinte de 6-14 semaines.

5. Méthode selon la revendication 2, dans laquelle le marqueur métabolique est normalisé par rapport à l'osmolalité ou à la créatinine urinaire.

6. Méthode selon la revendication 1, de criblage de la prédisposition au diabète sucré gestationnel chez une patiente, la patiente étant enceinte de 6-17 semaines, la méthode comprenant :

la mesure de la quantité d'au moins deux marqueurs métaboliques présents dans un échantillon à tester d'urine obtenu à partir de la patiente ;
la comparaison de la quantité du marqueur métabolique présent dans l'échantillon à tester avec un échantillon témoin ;
l'identification d'une patiente comme étant prédisposée au diabète, si la quantité des marqueurs présents dans l'échantillon à tester est accrue ou réduite par rapport à l'échantillon témoin ;
dans laquelle les marqueurs métaboliques comprennent de la 1-méthylhistidine et au moins un marqueur choisi dans le groupe constitué par l'alpha-CEHC glucuronide, l'ansérine, le méthylsuccinate, le xylonate, la tiglyl-carnitine, le quinolinate, la Pro-hydroxyPro, le 2-hydroxyisobutyrate, la N-acétylthréonine, la carnitine, et la trigonelline ;
où la concentration du ou de chaque marqueur métabolique est normalisée par rapport à un marqueur sélectionné de normalisation, choisi parmi la créatinine ou l'osmolalité.

7. Méthode selon la revendication 6, dans laquelle au moins trois marqueurs sont mesurés.

8. Méthode selon l'une quelconque des revendications 1 à 5 ou 7, dans laquelle le marqueur comprend de la 1-méthylhistidine et une combinaison quelconque d'un ou plusieurs marqueurs choisis dans le groupe constitué par l'alpha-CEHC glucuronide, l'ansérine, le méthylsuccinate, le xylonate, la tiglyl-carnitine, le quinolinate, la Pro-hydroxyPro, le 2-hydroxyisobutyrate, la N-acétylthréonine, la carnitine, la trigonelline, le 3-fucosyl-lactose, le 3-(3-hydroxyphényl)propionate, l'itaconate, et le saccharose.

9. Méthode selon l'une quelconque des revendications 1 à 5 ou 7, dans laquelle la combinaison de marqueurs comprend :

du saccharose et de la 1-méthylhistidine ;
du méthylsuccinate, de l'ansérine, et de la 1-méthylhistidine ;
du méthylsuccinate, du saccharose, et de la 1-méthylhistidine ;
du saccharose, de l'ansérine, et de la 1-méthylhistidine ; et

du méthylsuccinate, du saccharose, de l'ansérine, et de la 1-méthylhistidine.

10. Méthode selon l'une quelconque des revendications 1 à 5 ou 7, dans laquelle la combinaison de marqueurs comprend : de l'adipate, du méthylsuccinate, de la pyroglutamine, de la cytidine, de la 1-méthylhistidine, du 2-hydroxyisobutyrate, de la cystathionine, du saccharose et de l'ansérine.

11. Méthode selon l'une quelconque des revendications 1 à 5 ou 7, dans laquelle la combinaison de marqueurs comprend : de l'adipate, du méthylsuccinate, de la pyroglutamine, de la cytidine, de la 1-méthylhistidine, du 2-hydroxyisobutyrate, de la cystathionine, du saccharose, du glutamate, du galactose, du 3-hydroxy-3-méthylglutarate, et de l'ansérine.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012197539 A

**Non-patent literature cited in the description**

- *Obstet Gynecol,* 2013, vol. 122, 358-370
- **BRODY et al.** *Obstet Gynecol,* 2003, vol. 101, 380-392
- **BLAYO.** *Diabetes Metab,* 2004, vol. 30, 575-580
- **SACKS et al.** *Am J Obstet Gynecol,* 1989, vol. 161, 642-645
- **HANNA.** *Diabet Med,* 2002, vol. 19, 351-358
- **SMIRNAKIS et al.** *Am J Obstet Gynecol,* 2007, vol. 196, 410e1-410e7
- **GEORGIOU et al.** *Acta Diabetol,* 2008, vol. 45, 157-165
- **KULAKSIZGLU S. et al.** *Gynecol Endocrinol,* 2013, vol. 29, 137-140
- **WANG et al.** *Clinica Chimica Acta,* 2012, vol. 413 (1-2), 131-138
- **DIAZ et al.** *Journal of Proteome Research,* 2013, vol. 12 (6), 2946-2957